# EUROPEAN PATENT SPECIFICATION

(11) **EP 1 900 808 B1**
(45) Date of publication and mention of the grant of the patent: **05.03.2014**
(21) Application number: 07114681.5
(22) Date of filing: 21.08.2007
(51) Int. Cl.: C12N 1/02, B01D 61/48

(54) **Method of and apparatus for separating microorganisms from sample using electrodialysis and microorganism capturing means**
Verfahren und Vorrichtung zur Separierung von Mikroorganismen von Proben durch Elektrodialyse und Mittel zur Separierung von Mikroorganismen
Procédé et appareil pour la séparation de micro-organismes d'un échantillon utilisant l'électrodialyse et moyens pour la capture de micro-organismes

(30) Priority: 25.09.2006 KR 20060092920; 21.08.2006 KR 20060079056; 21.08.2006 KR 20060079053; 21.08.2006 KR 20060079054; 21.08.2006 KR 20060079055
(43) Date of publication of application: 19.03.2008
(73) Proprietor: Samsung Electronics Co., Ltd., Suwon-si, Gyeonggi-do, 443-742 (KR)
(72) Inventor: Han, Jung-im, Gyeonggi-do (KR); Kim, Joon-ho, Gyeonggi-do (KR); Jeong, Sung-young, Gyeonggi-do (KR); Lee, Young-sun, Gyeonggi-do (KR); Hwang, Kyu-youn, Gyeonggi-do (KR); Lee, Hun-joo, Gyeonggi-do (KR)
(74) Representative: Grünecker, Kinkeldey, Stockmair & Schwanhäusser

(56) References cited:
- WO-A-96/25998
- US-A1- 2006 057 635
- SATO T ET AL: "BACTERICIDAL EFFECT OF AN ELECTRODIALYSIS SYSTEM ON ESCHERICHIA-COLI CELLS" BIOELECTROCHEMISTRY AND BIOENERGETICS, vol. 21, no. 1, 1989, pages 47-54, XP002467705 ISSN: 0302-4598

## Description

### BACKGROUND OF THE INVENTION

### 1. Field of the Invention

The present invention relates to a method of separating microorganisms from a sample using electrodialysis and a microorganism capturing means, and an apparatus used in the method for separating microorganisms.

### 2. Description of the Related Art

In general, methods of separating microorganisms include a centrifugation method and a filtration method. Furthermore, in a method of concentrating and separating desired cells, the desired cells are allowed to specifically bind to receptors or ligands attached to a surface of a support. For example, an affinity chromatography method includes allowing a sample containing cells to flow over a support to which antibodies capable of specifically binding to the cells are attached, thereby binding the cells to the antibodies, and washing out unbound cells.

Further, Korean Laid-open Patent Publication No. 2006-0068979 describes a cell separating system using ultrasound and traveling wave dielectrophoresis. The cell separation system includes a piezoelectric transducer which is connected to both ends of an upper glass substrate and may convert an electric signal externally input into a mechanical vibration so as to apply the mechanical vibration to the upper glass substrate; and electrodes which are arranged on a lower substrate parallel to the upper glass substrate, the number of the electrodes being N. Between the upper glass substrate and the lower substrate, a fluid containing cells is filled. Each of the electrodes is placed in a vertical direction perpendicular to a longitudinal direction of the piezoelectric transducer, and all N electrodes are arranged at regular intervals along the longitudinal direction of the piezoelectric transducer.

Thus, in the above methods, the ligands or receptors are immobilized on the solid substrate or specific cells are selectively concentrated or separated from a sample using an externally supplied driving force.

WO 96/25998 A discloses a method and an apparatus for the concentration of a microorganism from complex sample material comprising use of desalting means to reduce the conductivity of the sample followed by dielectrophoresis.

Sato, T. et al., "Bactericidal effect of an electrodialysis system on E.Coli cells", Bioelectrochemistry and Bioenergetics, vol. 21, no. 1, 1989, pages 47-54 relates to an electrodialysis system with both cation- and anion-exchange membranes employed as a disinfectant method for water treatment comprising an anode, a cathode as well as two cation-exchange membranes and two anion-exchange membranes alternatively arranged between the anode and the cathode.

However, a method or an apparatus for separating cells by using the properties of a solid support itself and the conditions of a liquid medium have not yet been reported. Furthermore, in a method of separating cells by using the properties of a solid support itself and the conditions of a liquid medium and removing materials from the sample using electrodialysis, materials preventing the cells from binding to the solid support have not been reported yet.

### SUMMARY OF THE INVENTION

The present invention provides a method of separating microorganisms from a sample using electrodialysis and a microorganism capturing means according to claim 1.

The present invention also provides an apparatus for separating microorganisms from a sample using electrodialysis and a microorganism capturing means, according to claim 18.

According to an aspect of the present invention, there is provided a method of separating microorganisms from a sample, comprising:
introducing the sample into a reaction chamber of an apparatus for controlling a concentration of salts, the apparatus comprising the reaction chamber defined between a cation exchange membrane and an anion exchange membrane; a first electrode chamber defined between the anion exchange membrane and a first electrode and containing an ion exchange medium; and a second electrode chamber defined between the cation exchange membrane and a second electrode and containing an ion exchange medium;
applying a voltage between the first electrode and the second electrode to electrodialyze the sample in the reaction chamber, thereby reducing the concentration of salts in the sample; and
allowing the sample having the reduced concentration of salts to contact a microorganism capturing means,
wherein the sample is a biological sample, and wherein the microorganism capturing means is a non-planar solid support selected from the group consisting of a solid support having a plurality of pillars on its surface, a solid support in the form of beads, and a solid support having a sieve structure with a plurality of pores in its surface that is made of a material which binds to microorganisms.

According to another aspect of the present invention, there is provided an apparatus for separating microorganisms from a sample containing microorganisms comprising:
a reaction chamber having a sample inlet and an outlet and defined between a cation exchange membrane and an anion exchange membrane;
a first electrode chamber defined between the anion exchange membrane and a first electrode and containing an ion exchange medium;
a second electrode chamber defined between the cation exchange membrane and a second electrode and containing an ion exchange medium; and
a container connected to the reaction chamber via the outlet of the reaction chamber and containing a microorganism capturing means, wherein the microorganism capturing means is a non-planar solid support selected from the group consisting of a solid support having a plurality of pillars on its surface, a solid support in the form of beads, and a solid support having a sieve structure with a plurality of pores in its surface that is made of a material which binds to microorganisms.

### BRIEF DESCRIPTION OF THE DRAWINGS

The above and other features and advantages of the present invention will become more apparent by describing in detail exemplary embodiments thereof with reference to the attached drawings in which:
FIG. 1 is a top view illustrating an apparatus for controlling a concentration of salts used in a method of or an apparatus for separating microorganisms from a sample according to an embodiment of the present invention;
FIG. 2 is a top view illustrating an apparatus for controlling a concentration of salts used in a method of or an apparatus for separating microorganisms from a sample according to another embodiment of the present invention;
FIG. 3 is a top view illustrating an apparatus for controlling a concentration of salts used in a method of or an apparatus for separating microorganisms from a sample according to another embodiment of the present invention;
FIG. 4 is a view illustrating a process of reducing the concentration of salts in a sample using the apparatus illustrated in FIG. 2;
FIG. 5 is a view illustrating a process of reducing the concentration of salts in a sample using the apparatus illustrated in FIG. 3;
FIG. 6 is a graph illustrating efficiencies of cell capture by a solid support having an array of pillars on its surface from a urine sample;
FIG. 7 is a graph illustrating efficiencies of cell capture by a solid support having an array of pillars on its surface at various dilution ratios and flow rates from a urine sample;
FIG. 8 is a graph illustrating a desalting ratio of the urine sample against a voltage;
FIG. 9 is a graph illustrating an effect of electrodialysis of urine samples on an efficiency of binding microorganisms to a solid support;
FIG. 10 is a graph illustrating an effect of electrodialysis of three types of urine samples on an efficiency of binding microorganisms to a solid support;
FIG. 11 is a graph illustrating a Ct value of polymerase chain reaction (PCR) products obtained by using electrodialyzed urine samples as templates against a direct voltage;
FIG. 12 is a graph illustrating a viability of cells against a direct voltage; and
FIG. 13 is a graph illustrating a pH and a concentration of salts in a mixture of the electrodialyzed urine sample with a 200 mM acetate buffer against the concentration of urine in an electrodialyzed urine sample.

### DETAILED DESCRIPTION OF THE INVENTION

Hereinafter, the present invention will be described in detail by explaining embodiments of the invention with reference to the attached drawings.

According to an embodiment of the present invention, there is provided a method of separating microorganisms from a sample. The method comprises introducing the sample into a reaction chamber of an apparatus for controlling a concentration of salts, the apparatus comprising the reaction chamber defined between a cation exchange membrane and an anion exchange membrane; a first electrode chamber defined between the anion exchange membrane and a first electrode and containing an ion exchange medium; and a second electrode chamber defined between the cation exchange membrane and a second electrode and containing an ion exchange medium; applying a voltage between the first electrode and the second electrode to electrodialyze the sample in the reaction chamber, thereby reducing the concentration of salts in the sample; and allowing the sample having the reduced concentration of salts to contact a microorganism capturing means, wherein the sample is a biological sample, and wherein the microorganism capturing means is a non-planar solid support selected from the group consisting of a solid support having a plurality of pillars on its surface, a solid support in the form of beads, and a solid support having a sieve structure with a plurality of pores in its surface that is made of a material which binds to microorganisms.

The apparatus for controlling a concentration of salts into which a sample is introduced will be explained with reference to the attached drawings.

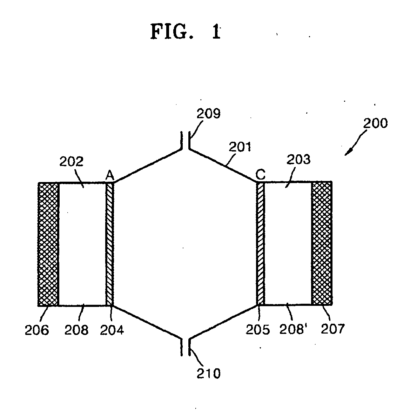

FIG. 1 is a top view illustrating an apparatus for controlling a concentration of salts used in a method according to an embodiment of the present invention.

Referring to FIG. 1, the apparatus 200 for controlling a concentration of salts comprises (a) a reaction chamber 201 defined between a cation exchange membrane 205 (C) and an anion exchange membrane 204 (A); (b) a first electrode chamber 202 defined between the anion exchange membrane 204 (A) and a first electrode 206 and containing an ion exchange medium 208; and (c) a second electrode chamber 203 defined between the cation exchange membrane 205 (C) and a second electrode 207 and containing an ion exchange medium 208'.

In the apparatus for controlling a concentration of salts, the reaction chamber 201 further comprises an inlet 209 and an outlet 210, through which a sample containing microorganisms flows in and out.

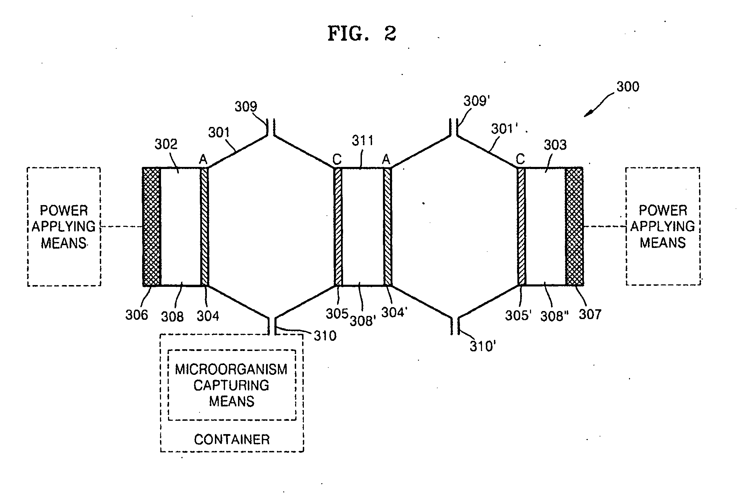

FIG. 2 is a top view illustrating an apparatus 300 for controlling a concentration of salts used in a method according to another embodiment of the present invention.

Referring to FIG. 2, the apparatus for controlling a concentration of salts comprises (a) a first reaction chamber 301 defined between a cation exchange membrane 305 (C) and an anion exchange membrane 304 (A); (b) a second reaction chamber 301' defined between a cation exchange membrane 305' (C) and an anion exchange membrane 304' (A); (c) an ion chamber 311 defined between the cation exchange membrane 305 (C) of the first reaction chamber 301 and the anion exchange membrane 304' (A) of the second reaction chamber 301' and containing an ion exchange medium 308'; (d) a first electrode chamber 302 defined between the anion exchange membrane 304 (A) of the first reaction chamber 301 and a first electrode 306 and containing an ion exchange medium 308; and (e) a second electrode chamber 303 defined between the cation exchange membrane 305' (C) of the second reaction chamber 301' and a second electrode 307 and containing an ion exchange medium 308".

That is, the apparatus for controlling a concentration of salts according to the current embodiment may comprise at least two reaction chambers 301 and 301' and the ion chamber 311 between the two reaction chambers 301 and 301', the ion chamber 311 being defined between the cation exchange membrane 305 and the anion exchange membrane 304' of the reaction chambers 301 and 301' and containing the ion exchange medium 308'.

The reaction chambers 301 and 301' further comprise inlets 309 and 309' and outlets 310 and 310', through which a sample containing microorganisms flows in and out.

In the apparatus for controlling a concentration of salts used in the method according to the current embodiment of the present invention, the reaction chamber is defined between the cation exchange membrane and the anion exchange membrane. The cation exchange membrane and the anion exchange membrane may respectively form at least two opposite sides of the reaction chamber. Alternatively, the cation exchange membrane and the anion exchange membrane may respectively form portions of at least two opposite sides of the reaction chamber.

In the apparatus for controlling a concentration of salts used in the method according to the current embodiment of the present invention, the first electrode chamber is defined between the anion exchange membrane and the first electrode and contains the ion exchange medium. That is, the first electrode chamber shares the anion exchange membrane with the reaction chamber, and the first electrode is provided in a portion or all of the side opposite to the anion exchange membrane. The ion exchange medium may be any ion conductive medium, and preferably an aqueous electrolytic solution.

In the apparatus for controlling a concentration of salts used in the method according to an embodiment of the present invention, the second electrode chamber is defined between the cation exchange membrane and the second electrode and contains the ion exchange medium. That is, the second electrode chamber shares the cation exchange membrane with the reaction chamber, and the second electrode is provided in a portion or all of the side opposite to the cation exchange membrane. The ion exchange medium may be any ion conductive medium, and preferably an aqueous electrolytic solution. The types of electrolytes are not specifically limited and may be readily selected by those skilled in the art such that they are suitable for the properties of the respective reactions in the reaction chamber.

The ion exchange medium contained in the first and second electrode chamber can be suitably selected by a person skilled in the art according the intended salt concentration of the biological sample. For example, the first and second electrode chamber can include a distilled deionised water as one example of an ion exchange medium, or a medium containing salts according to the desired salt concentraton of biological sample, the desired electrodialysis time and/or the pH of the biological sample. In one of the present Examples, 100mM Tris-HCI (pH 9.6) is used as ion exchange medium in the anode electrode chamber and distilled deionised water is used as ion exchange medium in the cathode electrode chamber.

In the apparatus for controlling a concentration of salts used in the method according to another embodiment of the present invention, the first electrode chamber may further comprise an ion chamber defined between the anion exchange membrane and a cation exchange membrane opposite to the anion exchange membrane. Furthermore, the second electrode chamber may further comprise an ion chamber defined between the cation exchange membrane and an anion exchange membrane opposite to the cation exchange membrane.

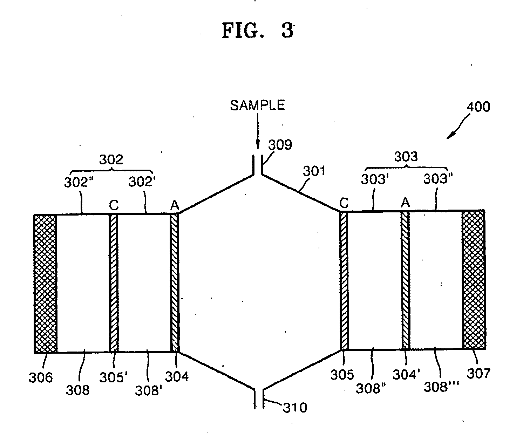

FIG. 3 is a top view illustrating an apparatus for controlling a concentration of salts used in a method according to still another embodiment of the present invention.

Referring to FIG. 3, the apparatus 400 for controlling a concentration of salts comprises (a) a reaction chamber 301 defined between a cation exchange membrane 305 (C) and an anion exchange membrane 304 (A); a first electrode chamber 302 consisting of a first ion chamber 302' and a second ion chamber 302", the first ion chamber 302' being defined between the anion exchange membrane 304 (A) and a cation exchange membrane 305' (C) and containing an ion exchange medium 308', and the second ion chamber 302" being defined the cation exchange membrane 305' (C) and a first electrode 306 and containing an ion exchange medium 308'; and a second electrode chamber 303 consisting of a first ion chamber 303' and a second ion chamber 303", the first ion chamber 303' being defined between the cation exchange membrane 305 (C) and an anion exchange membrane 304' (A) and containing an ion exchange medium 308", and the second ion chamber 303" being defined between the anion exchange membrane 304' (A) and a second electrode 307 and containing an ion exchange medium 308"'.

The reaction chamber 301 further comprises an inlet 309 and an outlet 310, through which the sample containing microorganisms flows in and out.

In the apparatus for controlling a concentration of salts used in the method of the present invention, cations can pass through the cation exchange membrane, but almost no anions can pass through the cation exchange membrane. Further, anions can pass through the anion exchange membrane, but almost no cations can pass through the anion exchange membrane. The cation exchange membrane and anion exchange membrane are well known in the art and may be easily obtained and used by those skilled in the art. For example, the cation exchange membrane may be a strong acid exchange membrane (having -SO₃-Na⁺; available from Nafion) or a weak acid exchange membrane (having -COO-Na⁺), and the anion exchange membrane may be a strong base exchange membrane (having -N⁺(CH₃)Cl⁻) or a weak base exchange membrane (having -N(CH₃)₂). Particularly, fluoride type ion exchange membrane such Nafion can includes perfluorocarbonsulfonic acid type polymer such as Nafion (registered trade mark, Dupont Co.), Aciplex (Asahi chemical Co.), Fremion (Asahi glass Co.) etc., polytrifluorostyrenesulfonic acid type polymer, perfluorocarbonphosphonic acid type polymer, trifluorostyrenesulfonic acid type polymer, ethylenetetrafluoroethylene-g-styrenesulfonic acid type polymer etc. Further, the examples of anion exchange membrane can be used in the present invention include A M 1, A M 2, A M 3, A M X, A M H, A F N (Tokuyama Corp) and AM P, A M V (Asahi glass Co.). The thickness of the cation or anion exchange membrane can be suitably selected by a person skilled in the art. When a size of reaction chamber is small, it is preferable that the thickness of the the cation or anion exchange membrane is as thin as possible since it is convenient to manufacture. Preferably, the thickness is 0.1 mm to 1.5mm.

In the apparatus for controlling a concentration of salts used in the method according to the present invention, the first electrode and the second electrode may be selected from the group consisting of platinum, gold, copper, and palladium, but they are not limited thereto.

In the method according to the present invention, a sample containing microorganisms is introduced into the reaction chamber of the apparatus for controlling a concentration of salts. The microorganisms include, but are not limited to, bacteria, fungi, and viruses. The sample may be introduced into the reaction chamber using any means well known in the art, for example, manually or by using a pump. Thus, the apparatus for controlling a concentration of salts may further comprise a pump for allowing the sample to flow into the reaction chamber, a pump for allowing the sample to flow out of the reaction chamber, and valves for controlling the inflow and outflow of the sample. For example, the pump for allowing the sample to flow in the reaction chamber may be connected to the inlet via channels, and the pump for allowing the sample to flow out from the reaction chamber may be connected to the outlet via channels. Alternatively, one pump can be used, which pump is suitable for allowing the sample to flow into and out of the reaction chamber.

The sample containing microorganisms may be a biological sample, preferably blood, urine, or saliva, and more preferably urine. In a method according to the present invention, the term "biological sample" refers to a sample comprising or consisting of cells or tissues, such as a biological fluid, isolated from a test subject. The test subject may include an animal or a human. Examples of the biological sample include saliva, sputum, blood, blood cells (such as, leukocytes and erythrocytes), amniotic fluid, serum, semen, bone marrow, a tissue or a microneedle biopsy specimen, urine, a peritoneal fluid, a pleural fluid, and cell cultures. Further examples of the biological sample include a tissue section, such as frozen tissue section for a histological purpose. The biological sample can have a various conducitivity according to the kinds of the sample. For example, the urine sample usually have a conducitivity of 7-30mS/cm.

The method according to the present invention includes applying a voltage between the first electrode and the second electrode to electrodialyze the sample in the reaction chamber, thereby reducing the concentration of salts in the sample. "Electrodialysis" is well known in the field to which the present invention pertains and refers to a method, wherein a sample containing salt is placed between membranes and an electric field is applied thereto. The cations and anions of the sample migrate in opposite directions and pass through the membranes, leaving purer water between the membrane filters. The first electrode and the second electrode may be connected to an anode power supply and a cathode power supply, respectively. The first electrode and the second electrode may be connected to a variable power supply, which can change the polarity of voltage. The direction of voltage connected to the first electrode and the second electrode may depend on an arrangement of the cation exchange membrane and the anion exchange membrane in the apparatus. Those skilled in the art may suitably select the direction of voltage such that a concentration of ions may be reduced in the reaction chamber.

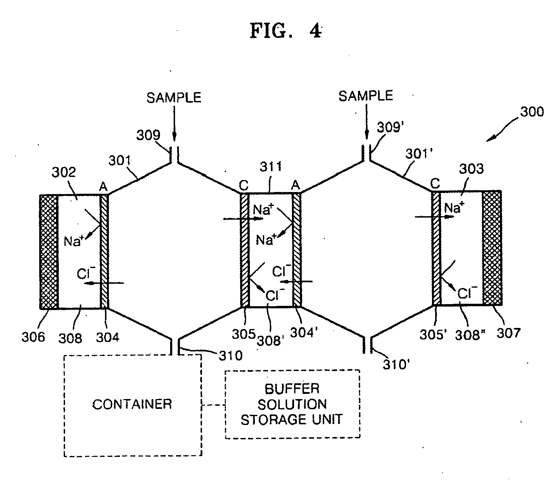

FIG. 4 is a view illustrating a process of reducing the concentration of salts in a sample using the apparatus illustrated in FIG. 2.

Referring to FIG. 4, a positive voltage is applied to the first electrode 306 and a negative voltage is applied to the second electrode 307. In this case, Na⁺ and Cl⁻ ions in the sample introduced into the first reaction chamber 301 and the second reaction chamber 301' move into the first electrode chamber 302, the ion chamber 311, or the second electrode chamber 303 through any of the cation exchange membranes 305 and 305' (C) and the anion exchange membrane 304 and 304' (A). Meanwhile, the Na⁺ and Cl⁻ ions in the ion exchange mediums 308, 308' and 308" of the first electrode chamber 302, the ion chamber 311, and the second electrode chamber 303 cannot move into the first reaction chamber 301 or the second reaction chamber 301' through any of the cation exchange membranes 305 and 305' (C) and the anion exchange membrane 304 and 304' (A).

As a result, while the Na⁺ and Cl⁻ ions are concentrated in the first electrode chamber 302, the ion chamber 311, and the second electrode chamber 303, they are diluted in the first reaction chamber 301 and the second reaction chamber 301'.

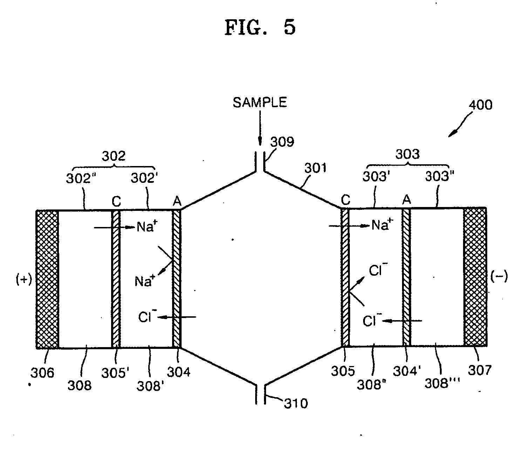

FIG. 5 is a view illustrating a process of reducing the concentration of salts in a sample using the apparatus illustrated in FIG. 3.

Referring to FIG. 5, the first electrode 306 is connected to an anode power supply and the second electrode 307 is connected to a cathode power supply, and then a voltage is applied across the reaction chamber 301 into which the sample is introduced. Cl⁻ ions in the sample flow into the first ion chamber 302' of the first electrode chamber 302 through the anion exchange membrane 304, but cannot pass through the cation exchange membrane 305', and thus, the Cl⁻ ions are concentrated in the first ion chamber 302'. Meanwhile, Na⁺ ions in the sample flow into the first ion chamber 303' of the second electrode chamber 303 through the cation exchange membrane 305, but cannot pass through the anion exchange membrane 304', and thus, the Na⁺ ions are concentrated in the first ion chamber 303'.

Consequently, the concentrations of the Na⁺ and Cl⁻ ions in the sample decrease. Each of the ion chambers 302', 302", 303' and 303" may comprise an electrolytic solution.

In the above electrodialysis, the concentrations of the Na⁺ and Cl⁻ ions in the sample decrease. In this way, substances which prevent microorganisms from attaching to a solid support, for example, salts, such as NaCl, and creatine can be removed from the sample. Substances which prevent a polymerase chain reaction (PCR) are also removed from the sample.

In the method according to the present invention, the sample having the reduced concentration of salts is allowed to contact a microorganism capturing means. The ion concentration of the sample has been decreased by electrodialysis.

The microorganism capturing means may be made of any material which binds to microorganisms. Examples of the material include, but are not limited to, a solid material, a semi-solid material, or a liquid material which binds to microorganisms. The microorganism capturing means is a non-planar solid support.

According to an exemplary embodiment of the present invention, there is provided a method of separating microorganisms from a sample, comprising introducing the sample into a reaction chamber of an apparatus for controlling a concentration of salts, the apparatus comprising the reaction chamber defined between a cation exchange membrane and an anion exchange membrane; a first electrode chamber defined between the anion exchange membrane and a first electrode and containing an ion exchange medium; and a second electrode chamber defined between the cation exchange membrane and a second electrode and containing an ion exchange medium; applying a voltage between the first electrode and the second electrode to electrodialyze the sample in the reaction chamber, thereby reducing the concentration of salts in the sample; and allowing the sample having the reduced concentration of salts to contact a non-planar solid support at a pH of 3.0-6.0, wherein the sample is a biological sample, wherein the non-planar solid support is selected from the group consisting of a solid support having a plurality of pillars on its surface, a solid support in the form of beads, and a solid support having a sieve structure with a plurality of pores in its surface that is made of a material which binds to microorganisms.

By allowing the sample having the reduced concentration of salts and a pH of 3.0-6.0 to contact the non-planar solid support, the microorganisms may attach to the non-planar solid support. It is assumed that since a surface area of the non-planar solid support is larger than that of a planar solid support and the use of the liquid medium having a pH of 3.0-6.0 denatures cell membranes of the microorganisms to decrease a solubility of the cell membranes in the liquid medium, a ratio of the microorganisms attached to the surface of the non-planar solid support relatively increases. However, the scope of the present invention is not limited to this specific mechanism.

According to the present invention, the microorganisms to be separated include bacteria, fungi, and viruses.

In the allowing of the sample having the reduced concentration of salts to contact the microorganism capturing means, the sample may be diluted with a solution capable of buffering the microorganisms at a low pH or a buffer. The buffer may be a phosphate buffer (such as, sodium phosphate, pH 3.0-6.0) or an acetate buffer (such as, sodium acetate, pH 3.0-6.0). The dilution ratio of the sample to the buffer may be 99:1-1:1,000, preferably 99:1-1:10, more preferably 99:1-1:4, but is not limited thereto.

In the allowing of the sample having the reduced concentration of salts to contact the microorganism capturing means, the sample may have salts in the concentration of 10-500 mM, preferably 50-300 mM. The sample may have ions selected from the group consisting of acetates and phosphates in the concentration of 10-500 mM, preferably 50-300 mM.

The non-planar solid support has a larger surface area than a planar solid support.

The non-planar solid support is selected from the group consisting of a solid support having a plurality of pillars on its surface, a solid support in the form of beads, and a solid support having a sieve structure with a plurality of pores on its surface. The non-planar solid support may be used alone or in an assembly of a plurality of solid supports (for example, an assembly filled in a tube or a container). The size of the bead is preferably 1 -100 µm in diameter. When the container is filled, it is preferable to filled more 40%. When a solid support has a sieve structure with a plurality of pores on its surface, the diameter of the pore is preferably in the range of 1 ~ 25µm.

Further, it is preferable that the solid support of the present inventon has surface to volume ration in the range of 100-300mm, but it is not limited thereto.

The non-planar solid support may be microchannels of a microfluidic device or inner walls of a microchamber. Thus, the method of separating microorganisms from a sample according to an embodiment of the present invention may be performed in a fluidic device or a microfluidic device having at least one inlet and outlet, the at least one inlet and outlet being in communication with each other through channels or microchannels.

As used herein, the term "microfluidic device" incorporates the concept of a microfluidic device that comprises microfluidic elements such as, e.g., microfluidic channels (also called microchannels or microscale channels). As used herein, the term "microfluidic" refers to a device component, e.g., chamber, channel, reservoir, or the like, that includes at lest one cross-sectional dimension, such as depth, width, length, diameter, etc. of from about 0.1 micrometer to about 1000 micrometer. Thus, the term "microchamber" and "microchannel" refer to a channel and a chamber that includes at lest one cross-sectional dimension, such as depth, width, and diameter of from about 0.1 micrometer to about 1000 micrometer, respectively.

In the method according to an exemplary embodiment of the present invention, in the allowing of the sample to contact the microorganism capturing means, the non-planar solid support may be a solid support having a plurality of pillars on its surface. A method of forming pillars on a solid support is well known in the art. For example, photolithography, or the like, used in manufacturing semiconductors may be used to form fine pillars on a solid support in high density. The pillars may have an aspect ratio, the height of the pillar : the length of the cross section of 1:1-20:1, but the aspect ratio is not limited thereto. The term "aspect ratio" used herein refers to a ratio of a height of a pillar to the length of a cross section of a pillar The length of a cross section of the pillar refers to a diameter when the shape of the cross section is a circle and it refers to an average value of the length of each side when the shape of the cross section is a rectangle. A ratio of the height of the pillars to a distance (height; distance) between the pillars may be 1:1-25:1. The distance between the pillars may be 5-100µm.

In the method according to the present invention, in the allowing of the sample having the reduced concentration of salts to contact the microorganism capturing means, the non-planar solid support may have hydrophobicity with a water contact angle of 70-95°. The hydrophobicity may be provided by coating a surface of the non-planar solid support with a compound selected from the group consisting of octadecyldimethyl (3-trimethoxysilyl propyl)ammonium (TMSAC), tridecafluorotetrahydrooctyltrimethoxysilane (FTEOS), CF₃(CF₂)₃CH₂CH₂SI(OCH₃) ₃, CF₃(CF₂)₅CH₂CH₂SI(OCH₃)₃, CF₃(CF₂)₇CH₂CH₂SI(OCH₃)₃, CF₃ (CF₂)₉CH₂CH₂SI(OCH₃)₃, (CF₃)₂CF(CF₂)₄CH₂CH₂SI(OCH₃)₃, (CF₃) ₂CF(CF₂)₆CH₂CH₂SI(OCH₃)₃, (CF₃)₂CF(CF₂)₈CH₂CH₂SI(OCH₃)₃, CF₃ (C₆H₄)C₂H₄Si(OCH₃)₃, CF₃(CF₂)₃(C₆H₄)C₂H₄Si(OCH₃)₃, CF₃ (CF₂)₅(C₆H₄)C₂H₄Si(OCH₃)₃, CF₃ (CF₂)₇(C₆H₄)C₂H₄S)(OCH₃)₃, CF₃ (CF₂) ₃CH₂CH₂SiCH₃(OCH₃)₂, CF₃(CF₂)₅CH₂CH₂SiCH₃(OCH₃)₂, CF₃ (CF₂)₇CH₂CH₂SiCH₃(OCH₃)₂, CF₃(CF₂)₉CH₂CH₂SiCH₃(OCH₃)₂, (CF₃) ₂CF(CF₂)₄CH₂CH₂SiCH₃(OCH₃)₂, (CF₃)₂CF(CF₂)₆CH₂CH₂SiCH₃(OCH₃)₂, (CF₃) ₂CF(CF₂)₈CH₂CH₂SiCH₃(OCH₃)₂, CF₃(C₆H₄)C₂H₄SiCH₃(OCH₃)₂, CF₃(CF₂)₃ (C₆H₄)C₂H₄SiCH₃(OCH₃)₂, CF₃(CF₂)₅(C₆H₄)C₂H₄SiCH₃(OCH₃)₂, CF₃ (CF₂)₇(C₆H₄)C₂H₄SiCH₃(OCH₃)₂, CF₃(CF₂)₃CH₂CH₂Si(OCH₂CH₃)₃, CF₃ (CF₂)₅CH₂CH₂Si(OCH₂CH₃)₃, and CF₃(CF₂)₇CH₂CH₂Si(OCH₂CH₃). For example, a SiO₂ layer of a solid support may be coated with a self-assembled monolayer (SAM) of a compound selected from the group consisting of TMSAC and FTEOS to provide a water contact angle of 70-95°.

In this application, the term "water contact angle" refers to water contact angle measured by a Kruss Drop Shape Analysis System type DSA 10 Mk2. A droplet of 1.5 µl deionized water is automatically placed on the sample. The droplet was monitored every 0.2 seconds for a period of 10 seconds by a CCD-camera and analyzed by Drop Shape Analysis software (DSA version 1.7, Kruss). The complete profile of the droplet was fitted by the tangent method to a general conic section equation. The angles were determined both at the right and left side. An average value is calculated for each drop and a total of five drops per sample are measured. The average of the five drops is taken the contact angle.

The non-planar solid support may have at least one amine-based functional group on its surface. The support may be coated with a compound selected from the group consisting of polyethyleneiminetrimethoxysilane (PEIM), aminopropyltriethoxysilane, N-(3-trimethoxysily)-propyl)ethylenediamine, and N-trimethoxysilylpropy-N,N,N-chloride trimethylammonium to provide a surface having the at least one amine-based functional group. For example, a SiO2 layer of a solid support may be coated with an SAM of PEIM. The amine-based functional group is positively charged at a pH of 3.0-6.0.

The non-planar solid support may be formed of any material which has a water contact angle of 70-95° or any material which has at least one amine-based functional group on its surface. Examples of the material include, but are not limited to, glass, silicon wafer, and plastics, etc. It is assumed that when the solid support having a surface having a water contact angle of 70-95° or a surface having at least one amine-based functional group on its surface contacts the sample containing microorganisms, the microorganisms may attach to the surface, but the scope of the present invention is not limited to this specific mechanism.

The method of separating microorganisms from a sample according to the present invention may further comprise washing out substances, other than the target microorganisms, which are not attached to the solid support after the allowing of the sample having the reduced concentration of salts to contact the microorganism capturing means. In the washing, any washing solution which does not separate the attached target microorganisms from the surface of the solid support and is capable of removing impurities which can adversely affect subsequent processes from the sample may be used. For example, an acetate buffer or a phosphate buffer used as a binding buffer, or the like, may be used. The washing solution may be a buffer having a pH of 3.0-6.0.

The term "separating microorganisms" used herein refers to concentrating of the microorganisms as well as isolation of pure microorganisms.

In the method according to the present invention, the microorganisms concentrated by the attachment to the solid support may be subjected to a subsequent process, such as isolation of DNAs. Alternatively, the microorganisms concentrated by the attachment to the solid support may be eluted from the solid support and then, the eluted microorganisms may be subjected to a subsequent process, such as isolation of DNAs.

Thus, the method of separating microorganisms from a sample according to the present invention may further comprise eluting the attached microorganisms from the solid support, after the allowing of the sample having the reduced concentration of salts to contact the microorganism capturing means and/or the washing. In the eluting of the microorganisms from the solid support, the eluting solution may be any solution known in the art which can detach the microorganisms from the solid support. Examples of the eluting solution include water and Tris buffer. The eluting solution may have a pH of 6.0 or greater.

According to another embodiment of the present invention, there is provided a method of treating microorganisms. The method of treating microorganisms comprises subjecting the microorganisms separated using the method of separating microorganisms from a sample according to the previous embodiment of the present invention to at least one process selected from the group consisting of isolation of nucleic acids, an amplification reaction of nucleic acids, and a hybridization reaction of nucleic acids, in a reaction chamber or a space other than the reaction chamber. The isolation of nucleic acids, the amplification reaction of nucleic acids, and the hybridization reaction of nucleic acids may be performed using any methods known in the art.

According to another embodiment of the present invention, there is provided an apparatus for separating microorganisms from a sample containing the microorganisms. The apparatus comprises a reaction chamber having a sample inlet and an outlet and defined between a cation exchange membrane and an anion exchange membrane; a first electrode chamber defined between the anion exchange membrane and a first electrode and containing an ion exchange medium; a second electrode chamber defined between the cation exchange membrane and a second electrode and containing an ion exchange medium; and a container connected to the reaction chamber via the outlet of the reaction chamber and containing a microorganism capturing means, wherein the microorganism capturing means is a non-planar solid support selected from the group consisting of a solid support having a plurality of pillars on its surface, a solid support in the form of beads, and a solid support having a sieve structure with a plurality of pores in its surface that is made of a material which binds to microorganisms.

The apparatus for separating microorganisms from a sample containing the microorganisms according to an embodiment of the present invention is characterized in that an apparatus for controlling a concentration of salts, comprising a reaction chamber having a sample inlet and an outlet and defined between a cation exchange membrane and an anion exchange membrane; a first electrode chamber defined between the anion exchange membrane and a first electrode and containing an ion exchange medium; and a second electrode chamber defined between the cation exchange membrane and a second electrode and containing an ion exchange medium, is connected to the container containing a microorganism capturing means via the outlet of the reaction chamber. The reaction chamber is in communication with the container.

With regard to the apparatus for separating microorganisms from a sample containing the microorganisms according to an embodiment of the present invention, the apparatus for controlling a concentration of salts will be explained with reference to the attached drawings.

FIG. 1 is a top view illustrating an apparatus for controlling a concentration of salts included in an apparatus for separating microorganisms from a sample containing microorganisms according to an embodiment of the present invention.

Referring to FIG. 1, the apparatus 200 for controlling a concentration of salts comprises (a) a reaction chamber 201 defined between a cation exchange membrane 205 (C) and an anion exchange membrane 204 (A); (b) a first electrode chamber 202 defined between the anion exchange membrane 204 (A) and a first electrode 206 and containing an ion exchange medium 208; and (c) a second electrode chamber 203 defined between the cation exchange membrane 205 (C) and a second electrode 207 and containing an ion exchange medium 208'.

In the apparatus for controlling a concentration of salts, the reaction chamber 201 further comprises an inlet 209 and an outlet 210, through which the sample containing microorganisms flows in and out.

FIG. 2 is a top view illustrating an apparatus for controlling a concentration of salts included in an apparatus for separating microorganisms from a sample containing microorganisms according to another embodiment of the present invention.

Referring to FIG. 2, the apparatus 300 for controlling a concentration of salts comprises (a) a first reaction chamber 301 defined between a cation exchange membrane 305 (C) and an anion exchange membrane 304 (A); (b) a second reaction chamber 301' defined between a cation exchange membrane 305' (C) and an anion exchange membrane 304' (A); (c) an ion chamber 311 defined between the cation exchange membrane 305 (C) of the first reaction chamber 301 and the anion exchange membrane 304' (A) of the second reaction chamber 301' and containing an ion exchange medium 308'; (d) a first electrode chamber 302 defined between the anion exchange membrane 304 (A) of the first reaction chamber 301 and a first electrode 306 and containing an ion exchange medium 308; and (e) a second electrode chamber 303 defined between the cation exchange membrane 305' (C) of the second reaction chamber 301' and a second electrode 307 and containing an ion exchange medium 308".

That is, the apparatus for controlling a concentration of salts may comprise at least two reaction chambers 301 and 301' and the ion chamber 311 between the two reaction chambers 301 and 301', the ion chamber 311 being defined between the cation exchange membrane 305 and the anion exchange membrane 304' of the reaction chambers 301 and 301' and containing the ion exchange medium 308'.

The reaction chambers 301 and 301' further comprise inlets 309 and 309' and outlets 310 and 310', through which the sample containing microorganisms flows in and out.

In the apparatus for controlling a concentration of salts included in the apparatus for separating microorganisms according to an embodiment of the present invention, the reaction chamber is defined between the cation exchange membrane and the anion exchange membrane. The cation exchange membrane and the anion exchange membrane may form at least two opposite sides of the reaction chamber. Alternatively, the cation exchange membrane and the anion exchange membrane may form portions of at least two opposite sides of the reaction chamber.

In the apparatus for controlling a concentration of salts included in the apparatus for separating microorganisms according to an embodiment of the present invention, the first electrode chamber is defined between the anion exchange membrane and the first electrode and contains the ion exchange medium. That is, the first electrode chamber shares the anion exchange membrane with the reaction chamber, and the first electrode is provided in a portion or all of the side opposite to the anion exchange membrane. The ion exchange medium may be any ion conductive medium, and preferably an aqueous electrolytic solution.

In the apparatus for controlling a concentration of salts included in the apparatus for separating microorganisms according to an embodiment of the present invention, the second electrode chamber is defined between the cation exchange membrane and the second electrode and contains the ion exchange medium. That is, the second electrode chamber shares the cation exchange membrane with the reaction chamber, and the second electrode is provided in a portion or all of the side opposite to the cation exchange membrane. The ion exchange medium may be any ion conductive medium, and preferably an aqueous electrolytic solution. The types of electrolytes are not specifically limited and may be readily selected by those skilled in the art such that they are suitable for the properties of the respective reactions.

In the apparatus for controlling a concentration of salts included in the apparatus for separating microorganisms according to an embodiment of the present invention, the first electrode chamber may further comprise an ion chamber defined between the anion exchange membrane and a cation exchange membrane opposite to the anion exchange membrane. Furthermore, the second electrode chamber may further comprise an ion chamber defined between the cation exchange membrane and an anion exchange membrane opposite to the cation exchange membrane.

FIG. 3 is a top view illustrating an apparatus for controlling a concentration of salts included in an apparatus for separating microorganisms from a sample containing microorganisms according to still another embodiment of the present invention.

Referring to FIG. 3, the apparatus 400 for controlling a concentration of salts comprises (a) a reaction chamber 301 defined between a cation exchange membrane 305 (C) and an anion exchange membrane 304 (A); a first electrode chamber 302 consisting of a first ion chamber 302' and a second ion chamber 302", the first ion chamber 302' being defined between the anion exchange membrane 304 (A) and a cation exchange membrane 305' (C) and containing an ion exchange medium 308', and the second ion chamber 302" being defined the cation exchange membrane 305' (C) and a first electrode 306 and containing an ion exchange medium 308'; and a second electrode chamber 303 consisting of a first ion chamber 303' and a second ion chamber 303", the first ion chamber 303' being defined between the cation exchange membrane 305 (C) and an anion exchange membrane 304' (A) and containing an ion exchange medium 308", and the second ion chamber 303" being defined the anion exchange membrane 304' (A) and a second electrode 307 and containing an ion exchange medium 308"'.

The reaction chamber 301 further comprises an inlet 309 and an outlet 310, through which the sample containing microorganisms flows in and out.

In the apparatus for controlling a concentration of salts included in an apparatus for separating microorganisms according to the present invention, cations can pass through the cation exchange membrane, but almost no anions can pass through the cation exchange membrane. Further, anions can pass through the anion exchange membrane, but almost no cations can pass through the anion exchange membrane. The cation exchange membrane and anion exchange membrane are well known in the art and may be easily obtained and used by those skilled in the art. For example, the cation exchange membrane may be a strong acid exchange membrane (having -SO₃-Na⁺; available from Nafion) or a weak acid exchange membrane (having -COO-Na⁺), and the anion exchange membrane may be a strong base exchange membrane (having -N⁺(CH₃)Cl⁻) or a weak base exchange membrane (having -N(CH₃)₂).

In the apparatus for controlling a concentration of salts included in an apparatus for separating microorganisms according to the present invention, the first electrode and the second electrode may be selected from the group consisting of platinum, gold, copper, and palladium, but they are not limited thereto.

The apparatus for controlling a concentration of salts may further comprise a pump for allowing the sample to flow into the reaction chamber, a pump for allowing the sample to flow out from the reaction chamber, and valves for controlling the inflow and outflow of the sample. For example, the pump for allowing the sample to flow into the reaction chamber may be connected to the inlet via channels, and the pump for allowing the sample to flow out from the reaction chamber may be connected to the outlet via channels. Alternatively, one pump can be used, which pump is suitable for allowing the sample to flow into and out of the reaction chamber.

The first electrode and the second electrode may be connected to an anode power supply and a cathode power supply, respectively. Alternatively, the first electrode and the second electrode may be connected to a cathode power supply and an anode power supply, respectively. The first electrode and the second electrode may be connected to a variable power supply, which can change the polarity of voltage. The polarity of voltage of the first electrode and the second electrode may depend on an arrangement of the cation exchange membrane and the anion exchange membrane in the apparatus. Those skilled in the art may suitably select the direction of voltage such that a concentration of ions may be reduced in the reaction chamber. Preferably, the voltage is 1V/mm to 3V/mm. A person skilled in the art can suitably select considering the desirable time of electrodialysis, membrane type and conductivity of biological sample used for example, urine.

The apparatus for controlling a concentration of salts further comprises a container connected to the reaction chamber via the outlet of the reaction chamber and containing a microorganism capturing means that is a microorganism capturing means is a non-planar solid support selected from the group consisting of a solid support having a plurality of pillars on its surface, a solid support in the form of beads, and a solid support having a sieve structure with a plurality of pores in its surface that is made of a material which binds to microorganisms.

Due to the property of the microorganism capturing means, the concentration of salts in the sample containing microorganisms, and the pH, or the like, the microorganisms may attach to a surface of the microorganism capturing means.

The microorganism capturing means may be any material which binds to microorganisms. Examples of the material include, but are not limited to, a solid material, a semi-solid material, or a liquid material which binds to microorganisms. The microorganism capturing means may be a non-planar solid support.

The non-planar solid support has a larger surface area than a planar solid support. The non-planar solid support may have a surface having an uneven structure. The term "uneven structure" used herein refers to a structure of which surface is not smooth and may have concaves and convexes. The uneven structure includes, but is not limited to, a surface having a plurality of pillars, a surface having a sieve structure with a plurality of pores, and a surface having a net-like structure.

The non-planar solid support may have any shape and may be selected from the group consisting of a solid support having a plurality of pillars on its surface, a solid support in the form of beads, and a solid support having a sieve structure with a plurality of pores on its surface. The non-planar solid support may be used alone or in an assembly of a plurality of the solid supports (for example, an assembly filled in a tube or a container).

The non-planar solid support may be microchannels of a microfluidic device or inner walls of a microchamber. Thus, the apparatus for separating microorganisms according to an embodiment of the present invention may be a fluidic device or a microfluidic device having at least one inlet and outlet, the at least one inlet and outlet being in communication with each other through channels or microchannels.

In the apparatus for separating microorganisms from a sample containing the microorganisms according to an embodiment of the present invention, the non-planar solid support may be a solid support having a plurality of pillars on its surface. A method of forming pillars on a solid support is well known in the art. For example, photolithography, or the like, used in manufacturing semiconductors may be used to form fine pillars on a solid support in high density. The pillars may have an aspect ratio of 1:1-20:1, but the aspect ratio is not limited thereto. The term "aspect ratio" used herein refers to a ratio of a diameter of a cross section of a pillar to a height of a pillar. A ratio of the height of the pillars to a distance between the pillars may be 1:1-25:1. The distance between the pillars may be 5-100µm.

In the apparatus for separating microorganisms from a sample containing microorganisms according to an embodiment of the present invention, the non-planar solid support may have hydrophobicity with a water contact angle of 70-95°. The hydrophobicity may be provided by coating a surface of the non-planar solid support with a compound selected from the group consisting of TMSAC and FTEOS. For example, a SiO₂ layer of a solid support may be coated with an SAM of a compound selected from the group consisting of TMSAC and FTEOS to provide a water contact angle of 70-95°.

The non-planar solid support may have at least one amine-based functional group on its surface. The support may be coated with PEIM to provide a surface having the at least one amine-based functional group. For example, an SiO₂ layer of a solid support may be coated with an SAM of PEIM.

The non-planar solid support may be formed of any material which has a water contact angle of 70-95° or any material which has at least one amine-based functional group on its surface. Examples of the material include, but are not limited to, glass, silicon wafer, and plastics, or the like. It is assumed that when the solid support having a surface having a water contact angle of 70-95° or a surface having at least one amine-based functional group on its surface contacts the sample containing microorganisms, the microorganisms may attach to the surface. However, the scope of the present invention is not limited to this specific mechanism.

In the apparatus for separating microorganisms from a sample containing the microorganisms according to the present invention, the container containing the solid support may be any container, for example, a container having the form of a chamber, a channel, or a column. In an exemplary embodiment of the present invention, a column container may be filled with a solid support in the form of beads.

The apparatus for separating microorganisms from a sample containing microorganisms according to the present invention may further comprise a buffer solution storage unit in or in fluidic communication with the container (e.g. see Fig. 4). The buffer solution storage unit contains a buffer which may be used to adjust the pH of the microorganism sample to 3.0-6.0 or dilute the microorganism sample. For example, the buffer may be a phosphate buffer or an acetate buffer. The buffer solution storage unit is in fluid communication with the container. Thus, the buffer solution storage unit may supply the buffer solution to the container, thereby providing a pH and a concentration of salts suitable for the sample solution from which the substances binding to the solid support have been removed by a desalting process in the reaction chamber.

The apparatus for separating microorganisms from a sample containing the microorganisms according to the present invention may be realized in a lab-on-a-chip.

Hereinafter, the present invention will be described in more detail with reference to the following examples. However, these examples are for illustrative purposes only and are not intended to limit the scope of the invention.

### EXAMPLES

### Example 1: Concentrating of E.coli cells in a urine mimetic solution using a solid support having an array of pillars on its surface - Screening of factors preventing capture of cells

In Example 1, a sample containing the bacteria cells was allowed to flow in a fluidic device including a chamber having an inlet and an outlet and having a surface of a silicon chip with a size of 10 mm x 23 mm on which an array of pillars is formed, thereby attaching the cells to the surface of the solid support. The number of the bacteria cells in the sample discharged from the fluidic device was determined using colony counting, and then an efficiency of capturing the bacteria cells by the solid support was calculated using the counted number of colonies. In the array of pillars, a distance between the pillars was 12 µm, a height of the pillars was 100 µm, and a cross section of each of the pillars was in the form of a square with a side of 25 µm. The ratio of surface to volume (V/S) is 152.18 mm, the number of pillars per mm2 is 816.

The array of pillars was formed on a SiO₂ layer coated with a self-assembled monolayer (SAM) of octadecyldimethyl (3-trimethoxysilyl propyl)ammonium (TMSAC). The water contact angle was about 80°.

Solutions based on a sodium acetate buffer and solutions based on dialyzed urine, both containing 0.01 OD₆₀₀ of *E*. *coli* cells, were used as the cell samples.

The solutions based on a sodium acetate buffer (pH 4.0) are as follows:
Buffer: a 100 mM sodium acetate buffer (pH 4.0),
Buffer + salt: a solution having the concentrations of the salts, 88 mM NaCl, 67 mM KCI, 38 mM NH₄Cl, and 18 mM Na₂SO₄, which was obtained by adding 0.514 g NaCl, 0.5 g KCI, 0.203 g NH₄Cl, and 0.259 g Na₂SO₄ to a 100 mM sodium acetate buffer (pH 4.0),
Buffer + salt + urea: a solution obtained by adding 333 mM urea to the "Buffer + salt" solution,
Buffer + salt + urea + creatine: a solution obtained by adding 333 mM urea and 9.8 mM creatine to the "Buffer + salt" solution,
Buffer + salt + urea + creatine + uric acid: a solution obtained by adding 333 mM urea, 9.8 mM creatine, and 2.5 mM uric acid to the "Buffer + salt" solution, and
Buffer + salt + urea + creatine + uric acid + glucose: a solution obtained by adding 333 mM urea, 9.8 mM creatine, 2.5 mM uric acid, and 0.6 mM glucose to the "Buffer + salt" solution.

The solutions based on dialyzed urine were obtained by mixing dialyzed urine and 2 x each of the solutions based on a sodium acetate buffer in ratio of 1:1 and a final pH of the solutions was 3.97.

200 µℓ of each of the samples was allowed to flow from the inlet to the outlet through the chamber at a flow rate of 200 µℓ/min. The experiments were repeated three times. The number of cells in each of the samples was counted before (Nᵢₙ) and after (Nₒᵤₜ) the flow through the chamber having the solid support and the efficiency of cell capture [%], (Nᵢₙ - Nₒᵤₜ)/Nᵢₙ)x100) was calculated.

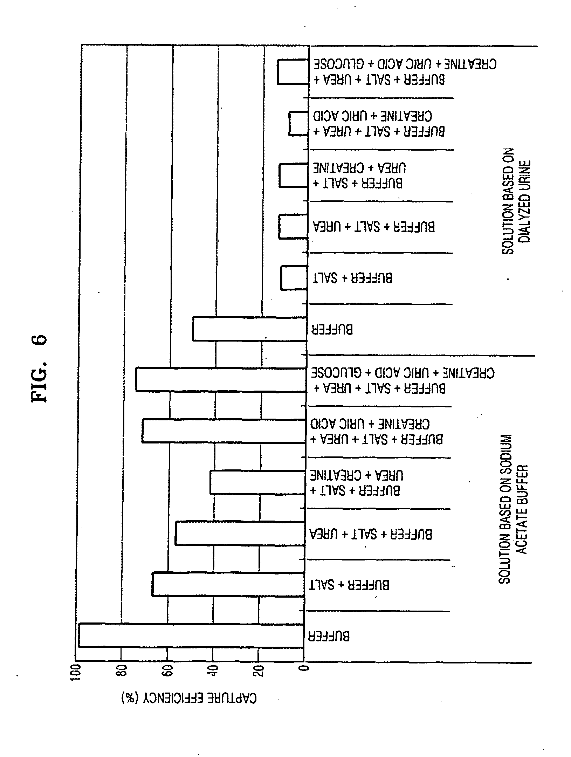

FIG. 6 is a graph illustrating efficiencies of cell capture by a solid support having an array of pillars on its surface from a urine sample. Referring to FIG. 6, when the salts and other substances (mainly creatine) were present in the samples, the efficiencies of cell capture decreased.

### Example 2: Concentrating of E. coli cells in a urine mimetic solution using a solid support having an array of pillars on its surface - Determination of effects of a dilution ratio of urine and a flow rate on the concentrating of the cells

In Example 2, a sample containing the bacteria cells was allowed to flow in a fluidic device including a chamber having an inlet and an outlet and having a surface of a silicon chip with a size of 10 mm x 23 mm on which an array of pillars is formed, thereby attaching the cells to the surface of the solid support. The number of the bacteria cells in the sample discharged from the fluidic device was determined using colony counting, and then an efficiency of capturing the bacteria cells by the solid support was calculated using the counted number of colonies. In the array of pillars, a distance between the pillars was 12 µm, a height of the pillars was 100 µm, and a cross section of each of the pillars was in the form of a square with a side of 25 µm. The ratio of surface to volume (V/S) is 152.18 mm, the number of pillars per mm2 is 816.

The array of pillars was formed on a SiO₂ layer coated with an SAM of polyethyleneiminetrimethoxysilane (PEIM). The water contact angle was about 80

Samples were prepared as follows. A buffer and urine were mixed in various dilution ratios to obtain a final volume of 1 ml. Then, 10 µℓ of 1.0 OD *E*. *coli* cells was added to each of the resulting mixtures.

200 µℓ of each of the diluted urine samples was allowed to flow from the inlet to the outlet through the chamber at a flow rate of 100 µℓ/min (F100), 300 µℓ/min (F300) and 500 µℓ/min (F500), respectively. The experiments were repeated three times. The number of cells in each of the samples was counted before and after the flow through the chamber having the solid support.

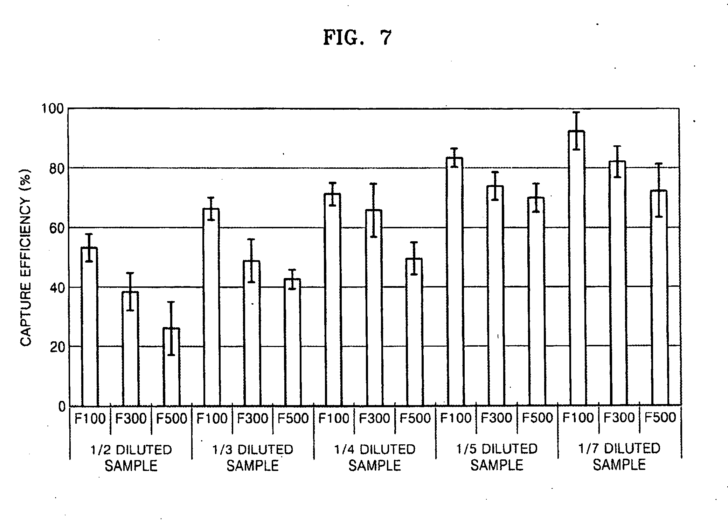

FIG. 7 is a graph illustrating efficiencies of cell capture by a solid support having an array of pillars on its surface at various dilution ratios and flow rates from a urine sample. Referring to FIG. 7, as the dilution ratio of the urine sample is increased, the efficiency of cell capture increases, and as the flow rate is increased, the efficiency of cell capture decreases.

Referring to FIGS. 6 and 7, since substances preventing the microorganisms from attaching to the solid support, such as ionic substances and creatine, etc. are present in the urine samples, they should be removed from the urine samples when it is intended to separate the microorganisms from urine using a solid support. Further, when the urine was diluted with a high dilution ratio of generally 5 or greater, the efficiency of capturing the microorganisms by the solid support was high.

### Example 3: Effects of voltage on desalting of a urine sample by electrodialysis

In Example 3, a urine sample was introduced into the reaction chamber 301 of the apparatus for controlling a concentration of salts illustrated in FIG. 3. The first electrode 306 and the second electrode 307 were connected to an anode power supply and a cathode power supply, respectively, and the urine sample was electrodialyzed.

The urine sample had a conductivity of 19.55 mS/cm and a pH of 6.5. Each of the ion chambers 302', 302", 303' and 303" contained 2.46 mS/cm NaCl aqueous solution as a conductive medium. The cation exchange membranes were made of a strong acid exchange membrane (-S03₃⁻Na⁺ form; available from Hankook Jungsoo Industries Co., Ltd., Ansan, Korea) and the anion exchange membranes were made of a strong base exchange membrane (-N⁺(CH₃)Cl⁻ form; available from Hankook Jungsoo Industries Co., Ltd., Ansan, Korea). The reaction chamber 301 had a dimension of 2 x 2 x 10 mm³. Pt electrodes having a size of 10 x 10 mm² were used and a distance between the electrodes was 1 cm.

0.2 ml of the urine sample was introduced into the reaction chamber 301 and a voltage of 1, 2, 3 and 4 V/mm was respectively applied between the electrodes for 3 minutes. Then, an electric conductivity of the urine sample in the reaction chamber was determined.

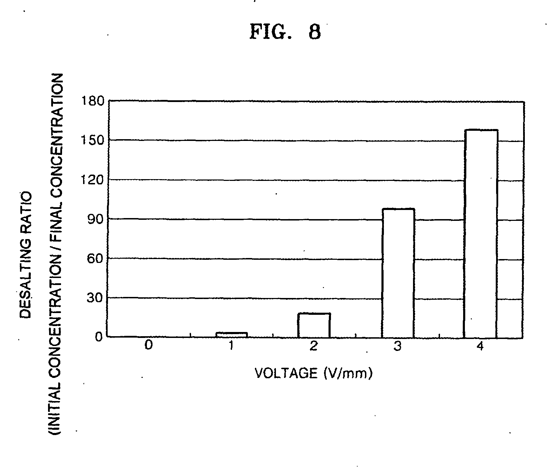

FIG. 8 is a graph illustrating a desalting ratio (the initial salt concentration divided by salt concentration in the electrodialyzed sample) of the urine sample against the voltage applied. Referring to FIG. 8, as the applied voltage is increased, the desalting ratio of the urine sample increases.

### Example 4: Effects of desalting of a urine sample by electrodialysis on microorganism capture by a solid support

In Example 4, a urine sample was desalted and the desalted urine sample was allowed to contact a solid support to determine an efficiency of capturing microorganisms by the solid support.

The urine sample was introduced into the reaction chamber 301 of the apparatus for controlling a concentration of salts illustrated in FIG. 3. The first electrode 306 and the second electrode 307 were connected to an anode power supply and a cathode power supply, respectively, and the urine sample was electrodialyzed.

A urine sample (urine sample 2) had a conductivity of 7.30 mS/cm and a pH of 6.5. Each of the ion chambers 302', 302", 303' and 303" contained 2.46 mS/cm NaCl aqueous solution as a conductive medium. The cation exchange membranes were made of a strong acid exchange membrane (-SO₃⁻Na⁺ form; available from Hankook Jungsoo Industries Co., Ltd., Ansan, Korea) (thickness about 1 mm) and the anion exchange membranes were made of a strong base exchange membrane (-N⁺(CH₃)Cl⁻ form; available from Hankook Jungsoo Industries Co., Ltd., Ansan, Korea) (thickness about 1 mm). The reaction chamber 301 had a dimension of 2 x 2 x 10 mm³. Pt electrodes having a size of 10 x 10 mm² were used and a distance between the electrodes was 1 cm. 0.2 ml of the urine sample was introduced into the reaction chamber 301 and voltages were applied between the electrodes for a total of 3.5 minutes: 1.5 V/mm for 30 seconds, 2.5 V/mm for one minute, and 3 V/mm for 2 minutes.

*E. coli* cells were added to the electrodialyzed sample to obtain an OD value of 0.01 (about 10⁷ cells/ml) and the resultant sample was diluted in a dilution ratio of 2 (D2) and 5 (D5), respectively, with a buffer. Subsequently, the diluted sample was allowed to contact a non-planar solid support to attach the microorganisms to the non-planar solid support. A cell binding efficiency of each of the urine samples was determined.

The electrodialyzed urine sample containing *E*. *coli* cells was allowed to flow in a fluidic device including a chamber having an inlet and an outlet and having a surface of a silicon chip with a size of 10 mm x 23 mm on which an array of pillars was formed, thereby attaching the cells to the surface of the solid support. The number of the *E*. *coli* cells in the sample discharged from the fluidic device was determined by optical density determination, and then an efficiency of capturing the *E. coli* cells by the solid support was calculated using the determined number of the cells. In the array of pillars, a distance between the pillars was 12 µm, a height of the pillars was 100 µm, and a cross section of each of the pillars was in the form of a square with a side of 25 µm, The ratio of surface to volume (V/S) is 152.18 mm, the number of pillars per mm2 is 816.

The array of pillars was formed on a SiO₂ layer coated with an SAM of TMSAC. The water contact angle was about 80°.

The urine samples containing 0.01 OD₆₀₀ of *E*. *coli* cells were diluted with a 100 mM sodium acetate buffer (pH 4.0) in dilution ratios of 2 (sample : buffer = 1:1) (hereinafter, referred to as D2) and 5 (sample : buffer = 1:4) (hereinafter, referred to as D5), respectively. D2 and D5 had a pH of 4.0-4.5.

200 µℓ of each of the urine samples was allowed to flow from the inlet to the outlet through the chamber at a flow rate of 200 µℓ/min. The experiments were repeated three times. The number of cells in each of the urine samples was counted before and after the flow through the chamber having the solid support.

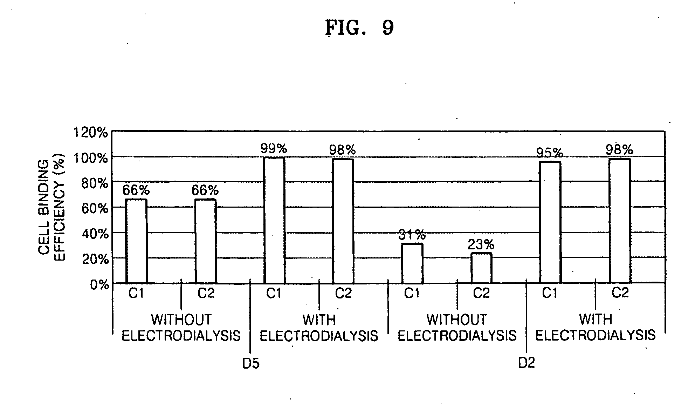

FIG. 9 is a graph illustrating an effect of electrodialysis of urine samples on the efficiency of binding microorganisms to a solid support.

Referring to FIG. 9, the electrodialyzed samples had remarkably greater cell binding efficiencies than the non-electrodialyzed samples (cell binding efficiency > 95%). In case of the electrodialyzed samples, both D5 and D2 had a cell binding efficiency of 95% or greater. However, in case of the non-electrodialyzed samples, D2 had a cell binding efficiency of less than 50%, which is remarkably lower than the cell binding efficiency of D5.

Further, urine samples 1, 2 and 3 having electric conductivities of 7.71 mS/cm, 7.30 mS/cm, and 11.2 mS/cm, respectively, and having a pH of 6.5-6.7 were electrodialyzed, diluted with a 100 mM sodium acetate buffer (pH 4.0) in a dilution ratio of 2 (hereinafter, referred to as D2), and then, allowed to pass through the fluidic device as above.

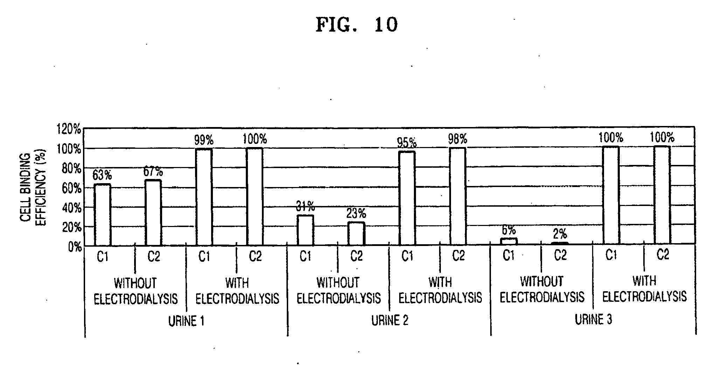

FIG. 10 is a graph illustrating an effect of electrodialysis of three types of urine samples on the efficiency of binding microorganisms to a solid support.

Referring to FIG. 10, the electrodialyzed samples had a cell binding efficiency of 95% or greater, although they were diluted in a dilution ratio of 2. However, the non-electrodialyzed samples had a cell binding efficiency of less than 70% and had a large deviation according to the type of the urine samples.

When urine samples 1, 2 and 3 were electrodialyzed and diluted in a dilution ratio of 5, they had a cell binding efficiency of 95% or greater (data are not shown).

### Example 5: Effects of electrodialysis on disruption and viability of microorganisms

A urine sample was introduced into the reaction chamber 301 of the apparatus for controlling a concentration of salts illustrated in FIG. 3. The first electrode 306 and the second electrode 307 were connected to an anode power supply and a cathode power supply, respectively, and the urine sample was electrodialyzed.

The urine sample had a conductivity of 7.30 mS/cm and a pH of 6.5. Each of the ion chambers 302', 302", 303' and 303" contained 2.46 mS/cm NaCl aqueous solution as a conductive medium. The cation exchange membranes were made of a strong acid exchange membrane (-SO₃⁻Na⁺ form; available from Hankook Jungsoo Industries Co., Ltd., Ansan, Korea) (thickness about 1 mm) and the anion exchange membranes were made of a strong base exchange membrane (-N⁺(CH₃)Cl⁻ form; available from Hankook Jungsoo Industries Co., Ltd., Ansan, Korea) (thickness about 1 mm). The reaction chamber 301 had a dimension of 2 x 2 x 10 mm³. Pt electrodes having a size of 10 x 10 mm² were used and a distance between the electrodes was 1 cm.

0.2 ml of the urine sample was introduced into the reaction chamber 301 and the respective voltages were applied between the electrodes for a total of 3.5 minutes.

*E. coli* cells were added to the electrodialyzed samples to obtain an OD value of 0.01 (about 10⁷ cells/ml). Then, the samples were centrifuged. A quantitative polymerase chain reaction (qPCR) was performed using the supernatant as a template and oligonucleotides having SEQ ID Nos. 1 and 2 as primers to confirm the effects of electrodialysis on disruption of cells. The qPCR was conducted using a Lightcycler^{™} 2.0 Real-Time PCR system from Roche Company, and the Ct value was calculated automatically by using the method stored in the Lightcycler^{™} 2.0 Real-Time PCR system. Further, the electrodialyzed urine samples were cultured and the number of colonies was counted to determine a viability of the microorganisms.

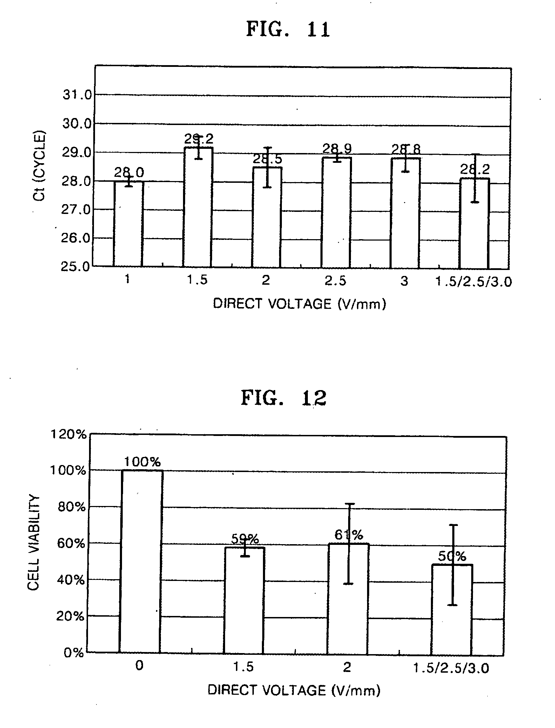

FIG. 11 is a graph illustrating a Ct value of PCR products obtained by using electrodialyzed urine samples as templates against a direct voltage.

Referring to FIG. 11, the Ct values did not deviate much when the applied voltage was changed. Thus, it is considered that the microorganisms were barely disrupted during the electrodialysis.

FIG. 12 is a graph illustrating a viability of cells against a direct voltage.

In FIG 12, the viability of cells was measured by counting the number of colonies after culturing the electrodialzyed urine sample. Referring to FIG. 12, as the applied voltage is increased, the viability of the microorganisms decreases.

### Example 6: Effects of dilution of electrodialyzed urine samples on a pH and a concentration of salts

A urine sample was electrodialyzed in the same manner as in Example 5 and the conductivity of the electrodialyzed urine sample was adjusted to 225 µS/cm using electrodialysis. Then, the resulting sample was diluted with a 200 mM acetate buffer (pH 4) and a pH and a concentration of salts of the diluted sample were determined.

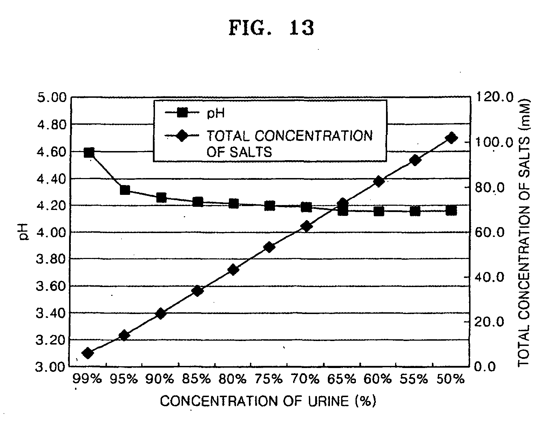

FIG. 13 is a graph illustrating a pH and a concentration of salts in a mixture of the electrodialyzed urine sample with a 200 mM acetate buffer against the concentration of urine in an electrodialyzed urine sample.

Referring to FIG. 13, when the concentration of the urine in the electrodialyzed urine sample was 55-99%, a pH value and a concentration of salts of the mixture were respectively included in ranges of pH 3-6 and 10-500 mM, which are optimal values for separating microorganisms using a solid support.

Thus, when the urine sample is electrodialyzed, the microorganisms may be separated from the urine sample although the urine sample is nearly diluted. As a result, an amount of the sample to be used in analysis may be reduced.

By using the method of separating microorganisms from a biological sample using electrodialysis according to the present invention, microorganisms, such as bacteria, fungi, or viruses may be efficiently separated from the biological sample.

Furthermore, by using the apparatus for separating microorganisms from a biological sample according to the present invention, microorganisms, such as bacteria, fungi, or viruses may be efficiently separated from the biological sample.

While the present invention has been particularly shown and described with reference to exemplary embodiments thereof, it will be understood by those of ordinary skill in the art that various changes in form and details may be made therein without departing from the scope of the present invention as defined by the following claims.
## Claims

1. A method of separating microorganisms from a sample, comprising:
introducing the sample into a reaction chamber of an apparatus for controlling a concentration of salts, the apparatus comprising the reaction chamber defined between a cation exchange membrane and an anion exchange membrane; a first electrode chamber defined between the anion exchange membrane and a first electrode and containing an ion exchange medium; and a second electrode chamber defined between the cation exchange membrane and a second electrode and containing an ion exchange medium;
applying a voltage between the first electrode and the second electrode to electrodialyze the sample in the reaction chamber, thereby reducing the concentration of salts in the sample; and
allowing the sample having the reduced concentration of salts to contact a microorganism capturing means,
wherein the sample is a biological sample, and
wherein the microorganism capturing means is a non-planar solid support selected from the group consisting of a solid support having a plurality of pillars on its surface, a solid support in the form of beads, and a solid support having a sieve structure with a plurality of pores in its surface that is made of a material which binds to microorganisms.

2. The method of claim 1, **characterized in** allowing the sample having the reduced concentration of salts to contact the microorganism capturing means at a pH of 3.0-6.0.

3. The method of claim 1 or 2, wherein the apparatus for controlling the concentration of salts comprises at least two reaction chambers and an ion chamber between the at least two reaction chambers, the ion chamber being defined between a cation exchange membrane and an anion exchange membrane of the reaction chambers and containing an ion exchange medium.

4. The method of claim 1 or 2, wherein the first electrode chamber comprises a first ion chamber and a second ion chamber, the first ion chamber being defined between the anion exchange membrane of the first electrode chamber and a cation exchange membrane which is positioned in the first electrode chamber and is opposite to the anion exchange membrane, and the second ion chamber being defined between the first electrode and the cation exchange membrane which is positioned in the first electrode chamber and is opposite to the anion exchange membrane; and
the second electrode chamber comprises a first ion chamber and a second ion chamber, the first ion chamber being defined between the cation exchange membrane of the second electrode chamber and an anion exchange membrane which is positioned in the second electrode chamber and is opposite to the cation exchange membrane, and the second ion chamber being defined between the second electrode and the anion exchange membrane which is positioned in the second electrode chamber and is opposite to the cation exchange membrane.

5. The method of claim 1 or 2, wherein the voltage is applied by connecting the first electrode and the second electrode to an anode power supply and a cathode power supply, respectively.

6. The method of claim 1 or 2, wherein the ion exchange medium is an aqueous electrolytic solution.

7. The method of claim 1 or 2, wherein the microorganisms are bacteria, fungi, or viruses.

8. The method of claim 1 or 2, wherein the biological sample is urine.

9. The method of claim 1, wherein the pillars have an aspect ratio of 1:1-20:1.

10. The method of claim 1, wherein a ratio of the height of the pillars to a distance between the pillars is 1:1-25:1.

11. The method of claim 1, wherein the distance between the pillars is 5-100µm.

12. The method of claim 2, wherein the non-planar solid support has a water contact angle of 70-95°.

13. The method of claim 12, wherein the water contact angle is provided by coating a surface of the non-planar solid support with octadecyldimethyl (3-trimethoxysilyl propyl)ammonium (TMSAC) or tridecafluorotetrahydrooctyltrimethoxysilane (FTEOS).

14. The method of claim 2, wherein the non-planar solid support has at least one amine-based functional group on its surface.

15. The method of claim 14, wherein the surface having the at least one amine-based functional group is coated with polyethyleneiminetrimethoxysilane (PEIM).

16. The method of claim 2, further comprising diluting the sample having the reduced concentration of salts with a phosphate buffer or an acetate buffer, prior to the allowing of the sample having the reduced concentration of salts to contact the microorganism capturing means.

17. A method of treating microorganisms, comprising:
separating microorganisms according to the method according to any one of claims 1 to 16 and subjecting the separated microorganism to at least one process selected from the group consisting of isolation of nucleic acids, an amplification reaction of nucleic acids, and a hybridization reaction of nucleic acids, in a reaction chamber or a space other than the reaction chamber.

18. An apparatus for separating microorganisms from a sample containing microorganisms comprising:
a reaction chamber having a sample inlet and an outlet and being defined between a cation exchange membrane and an anion exchange membrane;
a first electrode chamber defined between the anion exchange membrane and a first electrode and containing an ion exchange medium;
a second electrode chamber defined between the cation exchange membrane and a second electrode and containing an ion exchange medium; and
a container connected to the reaction chamber via the outlet of the reaction chamber and containing a microorganism capturing means, wherein the microorganism capturing means is a non-planar solid support selected from the group consisting of a solid support having a plurality of pillars on its surface, a solid support in the form of beads, and a solid support having a sieve structure with a plurality of pores in its surface that is made from a material which binds to microorganisms.

19. The apparatus of claim 18, comprising at least two reaction chambers and an ion chamber between the at least two reaction chambers, the ion chamber being defined between a cation exchange membrane and an anion exchange membrane of the reaction chambers and containing an ion exchange medium.

20. The apparatus of claim 18, wherein the first electrode chamber comprises a first ion chamber and a second ion chamber, the first ion chamber being defined between the anion exchange membrane of the first electrode chamber and a cation exchange membrane which is positioned in the first electrode chamber and is opposite to the anion exchange membrane, and the second ion chamber being defined between the first electrode and the cation exchange membrane which is positioned in the first electrode chamber and is opposite to the anion exchange membrane; and
the second electrode chamber comprises a first ion chamber and a second ion chamber, the first ion chamber being defined between the cation exchange membrane of the second electrode chamber and an anion exchange membrane which is positioned in the second electrode chamber and is opposite to the cation exchange membrane, and the second ion chamber being defined between the second electrode and the anion exchange membrane which is positioned in the second electrode chamber and is opposite to the cation exchange membrane.

21. The apparatus of claim 18, wherein the first electrode and the second electrode are connected to an anode power supply and a cathode power supply, respectively.

22. The apparatus of claim 18, wherein the container further comprises a buffer solution storage unit.

23. The apparatus of claim 18, wherein the pillars have an aspect ratio of 1:1-20:1.

24. The apparatus of claim 18, wherein a ratio of the height of the pillars to a distance between the pillars is 1:1-25:1.

25. The apparatus of claim 18, wherein the distance between the pillars is 5-1 00µM.

26. The apparatus of claim 18, wherein the non-planar solid support has a water contact angle of 70-95°.

27. The apparatus of claim 26, wherein the water contact angle is provided by coating a surface of the non-planar solid support with octadecyldimethyl (3-trimethoxysilyl propyl)ammonium (TMSAC) or tridecafluorotetrahydrooctyltrimethoxysilane (FTEOS).

28. The apparatus of claim 18, wherein the non-planar solid support has at least one amine-based functional group on its surface.

29. The apparatus of claim 28, wherein the surface having the at least one amine-based functional group is coated with polyethyleneiminetrimethoxysilane (PEIM).

## Patentansprüche

1. Verfahren zum Trennen von Mikroorganismen aus einer Probe, umfassend:
Einführen der Probe in eine Reaktionskammer einer Vorrichtung zur Steuerung einer Konzentration von Salzen, wobei die Vorrichtung die Reaktionskammer, die zwischen einer Kationen-Austauschmembran und einer Anionen-Austauschmembran definiert ist; eine erste Elektrodenkammer, die zwischen der Anionen-Austauschmembran und einer ersten Elektrode definiert ist und die ein Ionen-Austauschmedium enthält; und eine zweite Elektrodenkammer, die zwischen der Kationen-Austauschmembran und einer zweiten Elektrode definiert ist und die ein Ionen-Austauschmedium enthält, umfasst;
Anlegen einer Spannung zwischen der ersten Elektrode und der zweiten Elektrode, um die Probe in der Reaktionskammer zu elektrodialysieren, wodurch die Konzentration der Salze in der Probe verringert wird; und
Ermöglichen, dass die Probe mit der verringerten Konzentration an Salzen eine Mikroorganismen-Fangeinrichtung kontaktiert,
wobei die Probe eine biologische Probe ist, und
wobei die Mikroorganismen-Fangeinrichtung ein nicht-planarer fester Träger ist, gewählt aus der Gruppe bestehend aus einem festen Träger mit einer Vielzahl von Säulen auf dessen Oberfläche, einem festen Träger in der Form von Kugeln, und einem festen Träger mit einer Siebstruktur, mit einer Vielzahl von Poren in der Oberfläche, bestehend aus einem Material, welches Mikroorganismen bindet.

2. Verfahren nach Anspruch 1, **dadurch gekennzeichnet, dass** die Probe mit der verringerten Konzentration an Salzen die Mikroorganismen-Fangeinrichtung bei einem pH-Wert von 3,0 bis 6,0 kontaktieren kann.

3. Verfahren nach Anspruch 1 oder 2, wobei die Vorrichtung zur Steuerung der Konzentration an Salzen wenigstens zwei Reaktionskammern und eine Ionenkammer zwischen den wenigstens zwei Reaktionskammern umfasst, wobei die Ionenkammer zwischen einer Kationen-Austauschmembran und einer Anionen-Austauschmembran der Reaktionskammern definiert ist und ein Ionen-Austauschmedium enthält.

4. Verfahren nach Anspruch 1 oder 2, wobei die erste Elektrodenkammer eine erste lonenkammer und eine zweite Ionenkammer umfasst, wobei die erste Ionenkammer definiert ist zwischen der Anionenaustauschmembran der ersten Elektrodenkammer und einer Kationen-Austauschmembran, welche in der ersten Elektrodenkammer angeordnet ist und der Anionen-Austauschmembran gegenüberliegt, und die zweite Ionenkammer definiert ist zwischen der ersten Elektrode und der Kationen-Austauschmembran, welche in der ersten Elektrodenkammer angeordnet ist und der Anionen-Austauschmembran gegenüberliegt; und
die zweite Elektrodenkammer eine ersten Ionenkammer und eine zweite Ionenkammer umfasst, die erste Ionenkammer definiert ist zwischen der Kationen-Austauschmembran der zweiten Elektrodenkammer und einer Anionen-Austauschmembran, welche in der zweiten Elektrodenkammer angeordnet ist und der Kationen-Austauschmembran gegenüberliegt, und die zweite Ionenkammer definiert ist zwischen der zweiten Elektrode und der Anionen-Austauschmembran, welche in der zweiten Elektrodenkammer angeordnet ist und der Kationen-Austauschmembran gegenüberliegt.

5. Verfahren nach Anspruch 1 oder 2, wobei die Spannung angelegt wird, indem die erste Elektrode und die zweite Elektrode mit einer Anodenspannungsversorgung und einer Kathodenspannungsversorgung verbunden werden.

6. Verfahren nach Anspruch 1 oder 2, wobei das Ionen-Austauschmedium eine wässrige elektrolytische Lösung ist.

7. Verfahren nach Anspruch 1 oder 2, wobei die Mikroorganismen Bakterien, Pilze oder Viren sind.

8. Verfahren nach Anspruch 1 oder 2, wobei die biologische Probe Urin ist.

9. Verfahren nach Anspruch 1, wobei die Säulen ein Aspektverhältnis von 1:1 bis 20:1 aufweisen.

10. Verfahren nach Anspruch 1, wobei ein Verhältnis der Höhe der Säulen zu einem Abstand zwischen den Säulen 1:1 bis 25:1 beträgt.

11. Verfahren nach Anspruch 1, wobei der Abstand zwischen den Säulen 5 bis 100 µm beträgt.

12. Verfahren nach Anspruch 2, wobei der nicht-planare feste Träger einen Wasserkontaktwinkel von 70 bis 95° aufweist.

13. Verfahren nach Anspruch 12, wobei der Wasserkontaktwinkel bereitgestellt wird, indem eine Oberfläche des nicht-planaren festen Trägers mit Octodecyldimethyl(3-trimethoxysilylpropyl)-ammonium (TMSAC) oder Tridecafluortetrahydrooctyltrimethoxysilan (FTEOS) bedeckt wird.

14. Verfahren nach Anspruch 2, wobei der nicht-planare feste Träger wenigstens eine auf Amin basierende funktionelle Gruppe auf seiner Oberfläche aufweist.

15. Verfahren nach Anspruch 14, wobei die Oberfläche mit der wenigstens einen auf Amin basierenden funktionellen Gruppe mit Polyethylenimintrimethoxysilan (PEIM) beschichtet wird.

16. Verfahren nach Anspruch 2, des weiteren umfassend das Verdünnen der Probe mit der verringerten Konzentration an Salzen mit einem Phosphatpuffer oder einem Acetatpuffer, bevor die Probe mit der verringerten Konzentration an Salzen die Mikroorganismen-Fangeinrichtung kontaktieren kann.

17. Verfahren zum Behandeln von Mikroorganismen umfassend:
Trennen von Mikroorganismen gemäß des Verfahrens nach einem der Ansprüche 1 bis 16 und Unterwerfen des abgetrennten Mikroorganismus wenigstens einem Verfahren gewählt aus der Gruppe bestehend aus Isolation von Nukleinsäuren, einer Amplifikationsreaktion der Nukleinsäuren und einer Hybridisierungsreaktion der Nukleinsäuren in einer Reaktionskammer oder einem anderen Raum als der Reaktionskammer.

18. Vorrichtung zur Abtrennung von Mikroorganismen aus einer Probe enthaltend Mikroorganismen umfassend:
eine Reaktionskammer mit einem Probeneinlass und einem Probenauslass und definiert zwischen einer Kationen-Austauschmembran und einer Anionen-Austauschmembran;
eine erste Elektrodenkammer definiert zwischen der Anionen-Austauschmembran und einer ersten Elektrode und enthaltend ein Ionen-Austauschmedium;
eine zweite Elektrodenkammer definiert zwischen der Kationen-Austauschmembran und einer zweiten Elektrode und enthaltend ein Ionen-Austauschmedium; und
einen Behälter, der mit der Reaktionskammer über den Auslass der Reaktionskammer verbunden ist und der eine Mikroorganismen-Fangeinrichtung enthält, wobei die Mikroorganismen-Fangeinrichtung ein nicht-planarer fester Träger ist, gewählt aus der Gruppe bestehend aus einem festen Träger mit einer Vielzahl von Säulen auf dessen Oberfläche, einem festen Träger in der Form von Kugeln und einem festen Träger mit einer Siebstruktur, mit einer Vielzahl von Poren in der Oberfläche, bestehend aus einem Material, das Mikroorganismen bindet.

19. Vorrichtung nach Anspruch 18, umfassend wenigstens zwei Reaktionskammern und eine Ionenkammer zwischen den wenigstens zwei Reaktionskammern, wobei die Ionenkammer zwischen einer Kationen-Austauschmembran und einer Anionen-Austauschmembran der Reaktionskammern definiert ist und ein Ionen-Austauschmedium enthält.

20. Vorrichtung nach Anspruch 18, wobei die erste Elektrodenkammer eine erste lonenkammer und eine zweite Ionenkammer umfasst, wobei die erste Ionenkammer definiert ist zwischen der Anionen-Austauschmembran der ersten Elektrodenkammer und einer Kationen-Austauschmembran, welche in der ersten Elektrodenkammer angeordnet ist und der Anionen-Austauschmembran gegenüberliegt, und die zweite Ionenkammer definiert ist zwischen der ersten Elektrode und der Kationen-Austauschmembran, welche in der ersten Elektrodenkammer angeordnet ist und der Anionen-Austauschmembran gegenüberliegt; und
die zweite Elektrodenkammer eine erste Ionenkammer und eine zweite Ionenkammer umfasst, die erste Ionenkammer definiert ist zwischen der Kationen-Austauschmembran der zweiten Elektrodenkammer und einer Anionen-Austauschmembran, welche in der zweiten Elektrodenkammer angeordnet ist und der Kationen-Austauschmembran gegenüberliegt, und die zweite Ionenkammer definiert ist zwischen der zweiten Elektrode und der Anionen-Austauschmembran, welche in der zweiten Elektrodenkammer positioniert ist und der Kationen-Austauschmembran gegenüberliegt.

21. Vorrichtung nach Anspruch 18, wobei die erste Elektrode und die zweite Elektrode mit einer Anodenspannungsversorgung und einer Kathodenspannungsversorgung verbunden sind.

22. Vorrichtung nach Anspruch 18, wobei der Behälter des Weiteren eine Pufferlösungsspeichereinheit umfasst.

23. Vorrichtung nach Anspruch 18, wobei die Säulen ein Aspektverhältnis von 1:1 bis 20:1 aufweisen.

24. Vorrichtung nach Anspruch 18, wobei ein Verhältnis der Höhe der Säulen zu einem Abstand zwischen den Säulen 1:1 bis 25:1 beträgt.

25. Vorrichtung nach Anspruch 18, wobei der Abstand zwischen den Säulen 5 bis 100 µm beträgt.

26. Vorrichtung nach Anspruch 18, wobei der nicht-planare feste Träger einen Wasserkontaktwinkel von 70 bis 95° aufweist.

27. Vorrichtung nach Anspruch 26, wobei der Wasserkontaktwinkel durch Beschichten einer Oberfläche des nicht-planaren festen Trägers mit Octadecyldimethyl(3-trimethoxysilylpropyl)-ammonium (TMSAC) oder Tridecafluortetrahydrooctyltrimethoxysilan (FTEOS) bereitgestellt ist.

28. Vorrichtung nach Anspruch 18, wobei der nicht-planare feste Träger wenigstens eine auf Amin basierende funktionelle Gruppe auf seiner Oberfläche aufweist.

29. Vorrichtung nach Anspruch 28, wobei die Oberfläche mit der wenigstens einen auf Amin basierenden funktionellen Gruppe mit Polyethylenimintrimethoxysilan (PEIM) beschichtet ist.

## Revendications

1. Procédé de séparation de micro-organismes à partir d'un échantillon, comprenant :
l'introduction de l'échantillon dans une chambre de réaction d'un appareil pour contrôler une concentration en sels, l'appareil comprenant la chambre de réaction définie entre une membrane d'échange de cations et une membrane d'échange d'anions ; une chambre de première électrode définie entre la membrane d'échange d'anions et une première électrode et contenant un milieu d'échange d'ions ; et une chambre de deuxième électrode définie entre la membrane d'échange de cations et une deuxième électrode et contenant un milieu d'échange d'ions ;
l'application d'une tension électrique entre la première électrode et la deuxième électrode pour effectuer une électrodialyse de l'échantillon dans la chambre de réaction, réduisant ainsi la concentration en sels dans l'échantillon ; et
la mise en contact de l'échantillon ayant la concentration réduite en sels avec un moyen de capture de micro-organismes,
dans lequel l'échantillon est un échantillon biologique, et
dans lequel le moyen de capture de micro-organismes est un substrat solide non planaire sélectionné parmi le groupe constitué par un substrat solide possédant une pluralité de piliers sur sa surface, un substrat solide formé de perles, et un substrat solide ayant une structure de tamis avec une pluralité de pores dans sa surface, qui est fabriquée à partir d'un matériau qui se lie aux micro-organismes.

2. Procédé selon la revendication 1, **caractérisé en ce qu'**il met l'échantillon ayant la concentration réduite en sels en contact avec le moyen de capture de micro-organismes à un pH compris entre 3,0 et 6,0.

3. Procédé selon la revendication 1 ou 2, dans lequel l'appareil pour contrôler la concentration en sels comprend au moins deux chambres de réaction et une chambre à ions entre lesdites au moins deux chambres de réaction, la chambre à ions étant définie entre une membrane d'échange de cations et une membrane d'échange d'anions des chambres de réaction et contenant un milieu d'échange d'ions.

4. Procédé selon la revendication 1 ou 2, dans lequel la chambre de première électrode comprend une première chambre à ions et une deuxième chambre à ions, la première chambre à ions étant définie entre la membrane d'échange d'anions de la chambre de première électrode et une membrane d'échange de cations qui est agencée dans la chambre de première électrode et fait face à la membrane d'échange d'anions, et la deuxième chambre à ions étant définie entre la première électrode et la membrane d'échange de cations qui est agencée dans la chambre de première électrode et fait face à la membrane d'échange d'anions ; et
la chambre de deuxième électrode comprend une première chambre à ions et une deuxième chambre à ions, la première chambre à ions étant définie entre la membrane d'échange de cations de la chambre de deuxième électrode et une membrane d'échange d'anions qui est agencée dans la chambre de deuxième électrode et fait face à la membrane d'échange de cations, et la deuxième chambre à ions étant définie entre la deuxième électrode et la membrane d'échange d'anions qui est agencée dans la chambre de deuxième électrode et fait face à la membrane d'échange de cations.

5. Procédé selon la revendication 1 ou 2, dans lequel la tension électrique est appliquée en raccordant respectivement la première électrode et la deuxième électrode à une alimentation d'anode et à une alimentation de cathode.

6. Procédé selon la revendication 1 ou 2, dans lequel le milieu d'échange d'ions est une solution électrolytique aqueuse.

7. Procédé selon la revendication 1 ou 2, dans lequel les micro-organismes sont des bactéries, des champignons ou des virus.

8. Procédé selon la revendication 1 ou 2, dans lequel l'échantillon biologique est de l'urine.

9. Procédé selon la revendication 1, dans lequel le rapport d'aspect des piliers est compris entre 1/1 et 20/1.

10. Procédé selon la revendication 1, dans lequel le rapport hauteur/espacement des piliers est compris entre 1/1 et 25/1.

11. Procédé selon la revendication 1, dans lequel la distance entre les piliers est comprise entre 5 et 100 µm.

12. Procédé selon la revendication 2, dans lequel le substrat solide non planaire présente un angle de contact avec l'eau compris entre 70° et 95°.

13. Procédé selon la revendication 12, dans lequel l'angle de contact avec l'eau est pourvu en revêtant une surface du substrat solide non planaire avec de l'octadécyldiméthyl(3-triméthoxysilylpropyl)ammonium (TMSAC) ou du tridécafluorotétrahydrooctyltriméthoxysilane (FTEOS).

14. Procédé selon la revendication 2, dans lequel le substrat solide non planaire possède au moins un groupe fonctionnel à base d'amine sur sa surface.

15. Procédé selon la revendication 14, dans lequel la surface possédant ledit au moins un groupe fonctionnel à base d'amine est revêtue de polyéthylèneiminetriméthoxysilane (PEIM).

16. Procédé selon la revendication 2, comprenant en outre la dilution de l'échantillon ayant la concentration réduite en sels avec un tampon de phosphate ou un tampon d'acétate, avant de mettre l'échantillon ayant la concentration réduite en sels en contact avec le moyen de capture de micro-organismes.

17. Procédé de traitement de micro-organismes, comprenant :
la séparation de micro-organismes conformément au procédé selon l'une quelconque des revendications 1 à 16 et la soumission des micro-organismes séparés à au moins un procédé sélectionné parmi le groupe constitué par l'isolement d'acides nucléiques, une réaction d'amplification d'acides nucléiques, et une réaction d'hybridation d'acides nucléiques, dans un espace ou une chambre de réaction autre que la chambre de réaction.

18. Appareil pour séparer des micro-organismes à partir d'un échantillon contenant des micro-organismes, comprenant :
une chambre de réaction possédant une entrée et une sortie d'échantillon et définie entre une membrane d'échange de cations et une membrane d'échange d'anions ;
une chambre de première électrode définie entre la membrane d'échange d'anions et une première électrode et contenant un milieu d'échange d'ions ;
une chambre de deuxième électrode définie entre la membrane d'échange de cations et une deuxième électrode et contenant un milieu d'échange d'ions ; et
un récipient raccordé à la chambre de réaction via la sortie de la chambre de réaction et contenant un moyen de capture de micro-organismes, dans lequel le moyen de capture de micro-organismes est un substrat solide non planaire sélectionné parmi le groupe constitué par un substrat solide possédant une pluralité de piliers sur sa surface, un substrat solide formé de perles, et un substrat solide ayant une structure de tamis avec une pluralité de pores dans sa surface, qui est fabriquée à partir d'un matériau qui se lie aux micro-organismes.

19. Appareil selon la revendication 18, comprenant au moins deux chambres de réaction et une chambre à ions entre lesdites au moins deux chambres de réaction, la chambre à ions étant définie entre une membrane d'échange de cations et une membrane d'échange d'anions des chambres de réaction et contenant un milieu d'échange d'ions.

20. Appareil selon la revendication 18, dans lequel la chambre de première électrode comprend une première chambre à ions et une deuxième chambre à ions, la première chambre à ions étant définie entre la membrane d'échange d'anions de la chambre de première électrode et une membrane d'échange de cations qui est agencée dans la chambre de première électrode et fait face à la membrane d'échange d'anions, et la deuxième chambre à ions étant définie entre la première électrode et la membrane d'échange de cations qui est agencée dans la chambre de première électrode et fait face à la membrane d'échange d'anions ; et
la chambre de deuxième électrode comprend une première chambre à ions et une deuxième chambre à ions, la première chambre à ions étant définie entre la membrane d'échange de cations de la chambre de deuxième électrode et une membrane d'échange d'anions qui est agencée dans la chambre de deuxième électrode et fait face à la membrane d'échange de cations, et la deuxième chambre à ions étant définie entre la deuxième électrode et la membrane d'échange d'anions qui est agencée dans la chambre de deuxième électrode et fait face à la membrane d'échange de cations.

21. Appareil selon la revendication 18, dans lequel la première électrode et la deuxième électrode sont raccordées respectivement à une alimentation d'anode et à une alimentation de cathode.

22. Appareil selon la revendication 18, dans lequel le récipient comprend en outre une unité de stockage de solution tampon.

23. Appareil selon la revendication 18, dans lequel le rapport d'aspect des piliers est compris entre 1/1 et 20/1.

24. Appareil selon la revendication 18, dans lequel le rapport hauteur/espacement des piliers est compris entre 1/1 et 25/1.

25. Appareil selon la revendication 18, dans lequel la distance entre les piliers est comprise entre 5 et 100 µm.

26. Appareil selon la revendication 18, dans lequel le substrat solide non planaire présente un angle de contact avec l'eau compris entre 70° et 95°.

27. Appareil selon la revendication 26, dans lequel l'angle de contact avec l'eau est pourvu en revêtant une surface du substrat solide non planaire avec de l'octadécyldiméthyl(3-triméthoxysilylpropyl)ammonium (TMSAC) ou du tridécafluorotétrahydrooctyltriméthoxysilane (FTEOS).

28. Appareil selon la revendication 18, dans lequel le substrat solide non planaire possède au moins un groupe fonctionnel à base d'amine sur sa surface.

29. Appareil selon la revendication 28, dans lequel la surface possédant ledit au moins un groupe fonctionnel à base d'amine est revêtue de polyéthylèneiminetriméthoxysilane (PEIM).


<110> samsung Electronics co., Ltd.
<120> Method of and apparatus for separating microorganisms from sample using electrodialysis and microorganisms capturing means
<130> EP49821FZ106pau
<140> not yet assigned
   <141> herewith
<150> KR 10-2006-0079053
   <151> 2006-08-21
<150> KR 10-2006-0079054
   <151> 2006-08-21
<150> KR 10-2006-0079055
   <151> 2006-08-21
<150> KR 10-2006-0079056
   <151> 2006-08-21
<160> 2
<170> KopatentIn 1.71
<210> 1
   <211> 20
   <212> DNA
   <213> Artificial sequence
<220>
   <223> primer
<220>
   <221> misc_feature
   <222> (1)...(1)
   <223> y = t/u or c
<220>
   <221> misc_feature
   <222> (5)...(5)
   <223> k = g or t/u
<400> 1
   yccakactcc tacgggaggc 20
<210> 2
   <211> 22
   <212> DNA
   <213> Artificial sequence
<220>.
   <223> primer
<220>
   <221> misc_feature
   <222> (11)...(12)
   <223> r = g or a
<400> 2
   gtattaccgc rrctgctggc ac 22